(19) Europäisches Patentamt
European Patent Office
Office européen des brevets

(11) Veröffentlichungsnummer: **0 630 896 A1**

(12) # EUROPÄISCHE PATENTANMELDUNG

(21) Anmeldenummer: **94108789.2**

(22) Anmeldetag: **08.06.94**

(51) Int. Cl.5: **C07D 471/04**, A61K 31/435, C07D 401/14, C07D 401/10, A61K 31/415, A61K 31/44

(30) Priorität: **21.06.93 DE 4320432**

(43) Veröffentlichungstag der Anmeldung:
**28.12.94 Patentblatt 94/52**

(84) Benannte Vertragsstaaten:
**AT BE CH DE DK ES FR GB GR IE IT LI LU MC NL PT SE**

(71) Anmelder: **BAYER AG**

**D-51368 Leverkusen (DE)**

(72) Erfinder: **Fey, Peter, Dr.**
**Am Eickhof 23**
**D-42111 Wuppertal (DE)**
Erfinder: **Krämer, Thomas, Dr.**
**Schneewittchenweg 37**
**D-42111 Wuppertal (DE)**
Erfinder: **Dressel, Jürgen, Dr.**
**Tuchstrasse 48**
**D-42477 Radevormwald (DE)**
Erfinder: **Hanko, Rudolf, Dr.**
**Wadsaum 25**
**D-45134 Essen (DE)**
Erfinder: **Hübsch, Walter, Dr.**
**Wildsteig 22**
**D-42113 Wuppertal (DE)**
Erfinder: **Müller, Ulrich, Dr.**
**Neuer Triebel 91**
**D-42111 Wuppertal (DE)**
Erfinder: **Müller-Gliemann, Matthias, Dr.**
**Laibacher Strasse 10**
**D-42697 Solingen (DE)**
Erfinder: **Beuck, Martin, Dr.**
**Trills 7**
**D-40699 Erkrath (DE)**
Erfinder: **Bischoff, Hilmar, Dr.**
**Am Rohm 78**
**D-42113 Wuppertal (DE)**
Erfinder: **Wohlfeil, Stefan, Dr.**
**Tucherweg 25**
**D-40724 Hilden (DE)**
Erfinder: **Denzer, Dirk, Dr.**
**Claudiusweg 7**
**D-42115 Wuppertal (DE)**
Erfinder: **Kazda, Stanislav, Prof. Dr.**
**Gellertweg 18**
**D-42115 Wuppertal (DE)**
Erfinder: **Stasch, Johannes-Peter, Dr.**
**Alfred-Nobel-Strasse 109**
**D-42651 Solingen (DE)**
Erfinder: **Knorr, Andreas, Dr.**
**Trillser Graben 10**
**D-40699 Erkrath (DE)**
Erfinder: **Zaiss, Siegfreid, Dr.**
**Farnweg 3**
**D-42113 Wuppertal (DE)**

(54) **Substituierte Mono- und Bipyridylmethylderivate als Angiotensin II Antagonist.**

(57) Die substituierten Mono- und Bipyridyl-Derivate werden durch Umsetzung von substituierten Pyridinhalogeniden mit Phenylboronsäuren hergestellt. Sie eignen sich als Wirkstoffe in Arzneimitteln zur Behandlung von arterieller Hypertonie und Atherosklerose.

EP 0 630 896 A1

Die Erfindung betrifft substituierte Mono- und Bipyridylmethylderivate, Verfahren zu ihrer Herstellung sowie ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oklapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Arylheteroarylalkyl substituierte Triazole und Imidazole sind aus den Publikationen EP 508 445, EP 503 393, EP 504 888 und US 5 128 327 als A II-Antagonisten bekannt.

Außerdem werden in der EP 518 033 A1 kondensierte Heterocyclen mit A II-antagonistischer Wirkung beschrieben.

Die vorliegende Erfindung betrifft substituierte Mono- und Bipyridylmethylderivate der allgemeinen Formel (I)

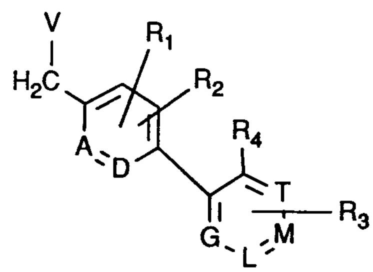

(I)

in welcher

| | |
|---|---|
| A, D, G, L, M und T | gleich oder verschieden sind und für die CH-Gruppe oder für ein Stickstoffatom stehen, wobei aber mindestens einer dieser Reste und maximal jeweils einer dieser Reste in jedem Cyclus für ein Stickstoffatom stehen darf, |
| V | für einen Rest der Formel |

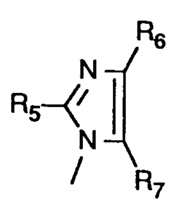

,

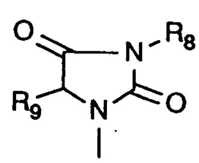

oder

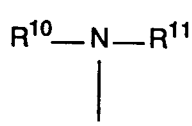

| | |
|---|---|
| | steht, worin |
| $R^5$, $R^8$ und $R^9$ | gleich oder verschieden sind und geradkettiges oder verzweigtes Alkyl mit bis zu 10 Kohlenstoffatomen bedeuten, das gegebenenfalls durch Cycloalkyl mit 3 bis 6 Kohlenstoffatomen, Hydroxy oder durch geradkettiges oder verzweigtes Alkoxy oder Alkylthio mit jeweils bis zu 6 Kohlenstoffatomen substituiert ist, oder Cycloalkyl mit 3 bis 6 Kohlenstoffatomen bedeuten, |
| $R^6$ | Halogen bedeutet, |
| $R^7$ | Formyl, Carboxy, geradkettiges oder verzweigtes Alkoxycarbonyl mit bis zu 6 Kohlenstoffatomen oder geradkettiges oder verzweigtes Alkyl mit bis zu 6 Koh- |

lenstoffatomen bedeutet, das gegebenenfalls durch Hydroxy substituiert ist,
oder

$R^6$ und $R^7$ unter Einbezug der Doppelbindung gemeinsam einen Pyridylring bilden, der 2- bis 3-fach gleich oder verschieden durch Carboxy, geradkettiges oder verzweigtes Alkoxycarbonyl mit bis zu 6 Kohlenstoffatomen oder durch geradkettiges oder verzweigtes Alkyl mit bis zu 6 Kohlenstoffatomen substituiert ist, das seinerseits durch Hydroxy substituiert sein kann,

$R^{10}$ geradkettiges oder verzweigtes Alkyl mit bis zu 8 Kohlenstoffatomen, Cycloalkyl mit 3 bis 6 Kohlenstoffatomen oder geradkettiges oder verzweigtes Acyl mit bis zu 8 Kohlenstoffatomen bedeutet, das gegebenenfalls durch geradkettiges oder verzweigtes Acyl mit bis zu 8 Kohlenstoffatomen substituiert ist,

$R^{11}$ Pyridyl bedeutet, das gegebenenfalls bis zu 2-fach gleich oder verschieden durch Carboxy, geradkettiges oder verzweigtes Alkoxycarbonyl mit bis zu 6 Kohlenstoffatomen oder durch geradkettiges oder verzweigtes Alkyl mit bis zu 6 Kohlenstoffatomen substituiert ist, das seinerseits durch Hydroxy substituiert sein kann, oder

geradkettiges oder verzweigtes Alkyl mit bis zu 8 Kohlenstoffatomen bedeutet, das gegebenenfalls durch Carboxy oder durch geradkettiges oder verzweigtes Alkoxycarbonyl mit bis zu 6 Kohlenstoffatomen substituiert ist, oder

eine Gruppe der Formel $-CO-NR^{12}R^{13}$ bedeutet,

worin

$R^{12}$ Wasserstoff oder Methyl bedeutet,

$R^{13}$ geradkettiges oder verzweigtes Alkyl mit bis zu 8 Kohlenstoffatomen bedeutet, das durch Carboxy oder geradkettiges oder verzweigtes Alkoxycarbonyl mit bis zu 6 Kohlenstoffatomen substituiert ist,

$R^1$, $R^2$ und $R^3$ gleich oder verschieden sind und für Wasserstoff, Halogen, Cyano, Nitro, Trifluormethyl, Amido oder für geradkettiges oder verzweigtes Alkyl oder Alkoxycarbonyl mit bis zu 6 Kohlenstoffatomen stehen,

$R^4$ für eine Gruppe der Formel $-CO-R^{14}$, $-SO_2R^{15}$, $-CO-NR^{16}R^{17}$, $-NH-SO_2R^{18}$ oder $-SO_2NR^{19}R^{20}$ steht,

worin

$R^{14}$ Hydroxy oder geradkettiges oder verzweigtes Alkoxy mit bis zu 6 Kohlenstoffatomen bedeutet,

$R^{15}$ Hydroxy, Trifluormethyl, geradkettiges oder verzweigtes Alkoxy oder Alkyl mit jeweils bis zu 6 Kohlenstoffatomen, Phenyl oder Benzyl bedeutet, die gegebenenfalls bis zu 2-fach gleich oder verschieden durch Halogen, Trifluormethyl oder geradkettiges oder verzweigtes Alkyl mit bis zu 4 Kohlenstoffatomen substituiert sind,

$R^{16}$ und $R^{17}$ gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,

oder

$R^{16}$ Wasserstoff bedeutet

und

$R^{17}$ die Gruppe $-SO_2R^{15}$ bedeutet,

worin

$R^{15}$ die oben angegebene Bedeutung hat,

$R^{18}$ die oben angegebene Bedeutung von $R^{15}$ hat und mit dieser gleich oder verschieden ist,

$R^{19}$ und $R^{20}$ die oben angegebene Bedeutung von $R^{16}$ und $R^{17}$ haben und mit diesen gleich oder verschieden sind,

oder

$R^{19}$ Wasserstoff oder geradkettiges oder verzweigtes Alkyl mit bis zu 4 Kohlenstoffatomen bedeutet

und

$R^{20}$ die oben angegebene Bedeutung von $R^{15}$ hat und mit dieser gleich oder verschieden ist,

oder

$R^4$ für einen Rest der Formel

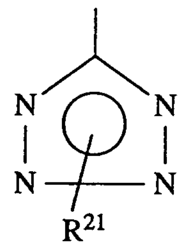

steht,
worin

$R^{21}$ Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,

und deren Salze.

Die erfindungsgemäßen substituierten Mono- und Bipyridylmethylderivate können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe oder einen Tetrazolylrest besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,

V für einen Rest der Formel

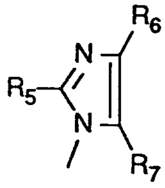

,

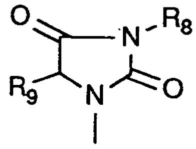

oder

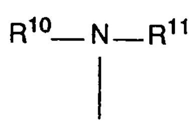

steht,
worin

| | |
|---|---|
| $R^5$, $R^8$ und $R^9$ | gleich oder verschieden sind und geradkettiges oder verzweigtes Alkyl mit jeweils bis zu 8 Kohlenstoffatomen bedeuten, das gegebenenfalls durch Cyclopropyl, Cyclopentyl, Cyclohexyl, Hydroxy oder durch geradkettiges oder verzweigtes Alkoxy oder Alkylthio mit jeweils bis zu 4 Kohlenstoffatomen substituiert ist, oder Cyclopropyl, Cyclopentyl oder Cyclohexyl bedeuten, |
| $R^6$ | Fluor, Chlor oder Brom bedeutet, |
| $R^7$ | Formyl, Carboxy, geradkettiges oder verzweigtes Alkoxycarbonyl mit bis zu 4 Kohlenstoffatomen oder geradkettiges oder verzweigtes Alkyl mit bis zu 4 Kohlenstoffatomen bedeutet, das gegebenenfalls durch Hydroxy substituiert ist, oder |
| $R^6$ und $R^7$ | unter Einbezug der Doppelbindung gemeinsam einen Pryridylring bilden, der 2-bis 3-fach gleich oder verschieden durch Carboxy, geradkettiges oder verzweigtes Alkoxycarbonyl mit bis zu 4 Kohlenstoffatomen oder durch geradkettiges oder verzweigtes Alkyl mit bis zu 4 Kohlenstoffatomen substituiert ist, das seinerseits durch Hydroxy substituiert sein kann, |
| $R^{10}$ | geradkettiges oder verzweigtes Alkyl mit bis zu 6 Kohlenstoffatomen, Cyclopropyl, Cyclopentyl, Cyclohexyl oder geradkettiges oder verzweigtes Acyl mit bis zu 6 Kohlenstoffatomen bedeutet, das gegebenenfalls durch geradkettiges oder verzweigtes Acyl mit bis zu 6 Kohlenstoffatomen substituiert ist, |
| $R^{11}$ | Pyridyl bedeutet, das gegebenenfalls bis zu 2-fach gleich oder verschieden durch Carboxy, geradkettiges oder verzweigtes Alkoxycarbonyl mit bis zu 4 Kohlenstoffatomen oder durch geradkettiges oder verzweigtes Alkyl mit bis zu 4 Kohlenstoffatomen substituiert ist, das seinerseits durch Hydroxy substituiert sein kann, oder |
| | geradkettiges oder verzweigtes Alkyl mit bis zu 6 Kohlenstoffatomen bedeutet, das gegebenenfalls durch Carboxy oder durch geradkettiges oder verzweigtes Alkoxycarbonyl mit bis zu 4 Kohlenstoffatomen substituiert ist,oder |
| | eine Gruppe der Formel $-CO-NR^{12}R^{13}$ bedeutet, |
| | worin |
| $R^{12}$ | Wasserstoff oder Methyl bedeutet, |
| $R^{13}$ | geradkettiges oder verzweigtes Alkyl mit bis zu 6 Kohlenstoffatomen bedeutet, das durch Carboxy oder geradkettiges oder verzweigtes Alkoxycarbonyl mit bis zu 4 Kohlenstoffatomen substituiert ist, |
| $R^1$, $R^2$ und $R^3$ | gleich oder verschieden sind und |
| | für Wasserstoff, Fluor, Chlor, Brom, Trifluormethyl, Amido oder für geradkettiges oder verzweigtes Alkyl oder Alkoxycarbonyl mit jeweils bis zu 4 Kohlenstoffatomen stehen, |
| $R^4$ | für eine Gruppe der Formel $-CO-R^{14}$, $-SO_2R^{15}$, $-CO-NR^{16}R^{17}$, $-NH-SO_2R^{18}$ oder $-SO_2NR^{19}R^{20}$ steht, |
| | worin |
| $R^{14}$ | Hydroxy oder geradkettiges oder verzweigtes Alkoxy mit bis zu 4 Kohlenstoffatomen bedeutet, |
| $R^{15}$ | geradkettiges oder verzweigtes Alkyl mit bis zu 4 Kohlenstoffatomen, Benzyl, Trifluormethyl oder p-Tolyl bedeutet, |
| $R^{16}$ und $R^{17}$ | gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist, |
| | oder |
| $R^{16}$ | Wasserstoff bedeutet |
| | und |
| $R^{17}$ | die Gruppe $-SO_2R^{15}$ bedeutet, |
| | worin |
| $R^{15}$ | die oben angegebene Bedeutung hat, |
| $R^{18}$ | die oben angegebene Bedeutung von $R^{15}$ hat und mit dieser gleich oder verschieden ist, |
| $R^{19}$ und $R^{20}$ | die oben angegebene Bedeutung von $R^{16}$ und $R^{17}$ haben und mit dieser gleich oder verschieden sind, |
| | oder |

$R^{19}$ Wasserstoff oder Methyl bedeutet,

$R^{20}$ die oben angegebene Bedeutung von $R^{15}$ hat und mit dieser gleich oder verschieden ist,

oder

$R^4$ für einen Rest der Formel

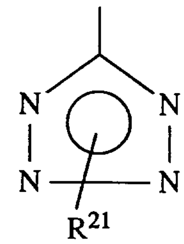

steht,

worin

$R^{21}$ Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,

und deren Salze.

Besonders bevorzugt sind Verbindungen der allgemeinen Formel (I),
in welcher

A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,

V für einen Rest der Formel

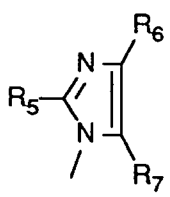 , 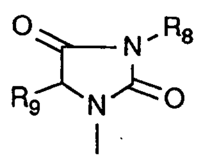 oder 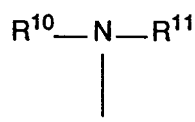

steht,

worin

$R^5$, $R^8$ und $R^9$ gleich oder verschieden sind und geradkettiges oder verzweigtes Alkyl mit bis zu 6 Kohlenstoffatomen bedeuten, das gegebenenfalls durch Cyclopropyl, Hydroxy, geradkettiges oder verzweigtes Alkoxy oder Alkylthio mit jeweils bis zu 3 Kohlenstoffatomen substituiert ist oder

Cyclopropyl, Chlor oder Iod bedeuten,

$R^6$ Fluor, Chlor oder Brom bedeutet,

$R^7$ Formyl, Carboxy, geradkettiges oder verzweigtes Alkoxycarbonyl mit bis zu 3 Kohlenstoffatomen oder geradkettiges oder verzweigtes Alkyl mit bis zu 4 Kohlenstoffatomen bedeutet, das gegebenenfalls durch Hydroxy substituiert ist,

oder

$R^6$ und $R^7$ unter Einbezug der Doppelbindung gemeinsam einen Pyridylring bilden, der 2- oder 3-fach gleich oder verschieden durch Carboxy, geradkettiges oder verzweigtes Alkoxycarbonyl mit bis zu 3 Kohlenstoffatomen oder durch geradkettiges oder verzweigtes Alkyl mit bis zu 3 Kohlenstoffatomen substituiert ist, das seinerseits durch Hydroxy substituiert sein kann,

$R^{10}$ geradkettiges oder verzweigtes Alkyl mit bis zu 5 Kohlenstoffatomen, Cyclopropyl, Cyclopentyl, Cyclohexyl oder geradkettiges oder verzweigtes Acyl mit bis zu 5 Kohlenstoffatomen bedeutet, das gegebenenfalls durch geradkettiges oder verzweigtes Acyl mit bis zu 5 Kohlenstoffatomen substituiert ist,

| | |
|---|---|
| $R^{11}$ | Pyridyl bedeutet, das gegebenenfalls bis zu 2-fach gleich oder verschieden durch Carboxy, geradkettiges oder verzweigtes Alkoxycarbonyl mit bis zu 3 Kohlenstoffatomen oder durch geradkettiges oder verzweigtes Alkyl mit bis zu 3 Kohlenstoffatomen substituiert ist, das seinerseits durch Hydroxy substituiert sein kann, oder geradkettiges oder verzweigtes Alkyl mit bis zu 5 Kohlenstoffatomen bedeutet, das gegebenenfalls durch Carboxy oder durch geradkettiges oder verzweigtes Alkoxycarbonyl mit bis zu 3 Kohlenstoffatomen substituiert ist,oder eine Gruppe der Formel $-CO-NR^{12}R^{13}$ bedeutet, worin |
| $R^{12}$ | Wasserstoff oder Methyl bedeutet, |
| $R^{13}$ | geradkettiges oder verzweigtes Alkyl mit bis zu 6 Kohlenstoffatomen bedeutet, das durch Carboxy oder geradkettiges oder verzweigtes Alkoxycarbonyl mit bis zu 3 Kohlenstoffatomen substituiert ist, |
| $R^1$, $R^2$ und $R^3$ | gleich oder verschieden sind und für Wasserstoff, Fluor, Chlor, Brom oder Methyl stehen, |
| $R^4$ | für eine Gruppe der Formel $-CO-R^{14}$, $-SO_2-R^{15}$, $-CO-NR^{16}R^{17}$, $-NH-SO_2R^{18}$ oder $-SO_2-NR^{19}R^{20}$ bedeutet, worin |
| $R^{14}$ | Hydroxy oder geradkettiges oder verzweigtes Alkoxy mit bis zu 3 Kohlenstoffatomen bedeutet, |
| $R^{15}$ | Methyl, Trifluormethyl, Benzyl oder p-Tolyl bedeutet, |
| $R^{16}$ und $R^{17}$ | gleich oder verschieden sind und Wasserstoff, Cyclopropyl, Cyclopentyl oder geradkettiges oder verzweigtes Alkyl mit bis zu 4 Kohlenstoffatomen bedeuten, das gegebenenfalls durch Phenyl substituiert ist, |
| $R^{16}$ | Wasserstoff bedeutet und |
| $R^{17}$ | die Gruppe $-SO_2-R^{15}$ bedeutet, worin |
| $R^{15}$ | die oben angegebene Bedeutung hat, |
| $R^{18}$ | die oben angegebene Bedeutung von $R^{15}$ hat und mit dieser gleich oder verschieden ist, |
| $R^{19}$ und $R^{20}$ | die oben angegebene Bedeutung von $R^{16}$ und $R^{17}$ haben und mit dieser gleich oder verschieden sind, oder |
| $R^{19}$ | Wasserstoff oder Methyl bedeutet und |
| $R^{20}$ | die oben angegebene Bedeutung von $R^{15}$ hat und mit dieser gleich oder verschieden ist oder |
| $R^4$ | für den Tetrazolylrest der Formel 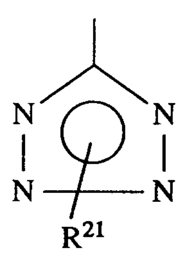 steht, worin |
| $R^{21}$ | Wasserstoff oder geradkettiges oder verzweigtes Alkyl mit bis zu 4 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet, |

und deren Salze.

Ganz besonders bevorzugt sind Verbindungen der allgemeinen Formel (I)
in welcher
A oder D für ein Stickstoffatom steht und der andere Rest jeweils für die CH-Gruppe steht,
G, L, M und T für die CH-Gruppe stehen,
V für einen Rest der Formel

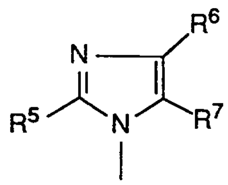 , 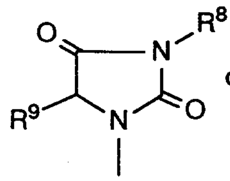 oder 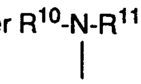

steht,
worin

| | |
|---|---|
| $R^5$, $R^8$ und $R^9$ | gleich oder verschieden sind und geradkettiges oder verzweigtes Alkyl mit bis zu 6 Kohlenstoffatomen oder Cyclopropyl bedeuten, |
| $R^6$ | Fluor, Chlor oder Brom bedeutet, |
| $R^7$ | Formyl, Carboxy, Methoxy- oder Ethoxycarbonyl oder Hydroxymethyl bedeutet, oder |
| $R^6$ und $R^7$ | unter Einbezug der Doppelbindung gemeinsam einen Pyridylrest bilden, der bis zu zweifach durch Brom oder Methyl substituiert sein kann, |
| $R^{10}$ | geradkettiges oder verzweigtes Alkyl mit bis zu 5 Kohlenstoffatomen bedeutet, oder geradkettiges oder verzweigtes Acyl mit bis zu 5 Kohlenstoffatomen bedeutet, das gegebenenfalls durch Acyl mit bis zu 5 Kohlenstoffatomen substituiert ist, |
| $R^{11}$ | Pyridyl bedeutet, das durch Carboxy oder Alkoxycarbonyl mit bis zu 3 Kohlenstoffatomen substituiert sein kann, oder geradkettiges oder verzweigtes Alkyl mit bis zu 5 Kohlenstoffatomen bedeutet, das durch Carboxy oder Alkoxycarbonyl mit bis zu 3 Kohlenstoffatomen substituiert sein kann, oder eine Gruppe der Formel $-CO-NR^{12}R^{13}$ bedeutet, worin |
| $R^{12}$ | Wasserstoff oder Methyl bedeutet |
| $R^{13}$ | geradkettiges oder verzweigtes Alkyl mit bis zu 6 Kohlenstoffatomen bedeutet, das durch Carboxy oder Alkoxycarbonyl mit bis zu 3 Kohlenstoffatomen substituiert ist, |
| $R^1$, $R^2$ und $R^3$ | für Wasserstoff stehen, |
| $R^4$ | für einen Tetrazolylrest der Formel |

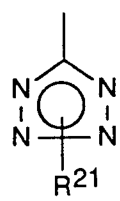

steht,
worin

| | |
|---|---|
| $R^{21}$ | Wasserstoff oder die Triphenylmethylgruppe bedeutet, |

und deren Salze.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II)

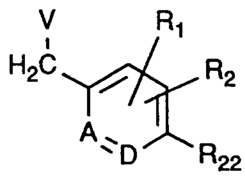

(II)

in welcher

A, D, V, $R^1$ und $R^2$ die oben angegebene Bedeutung haben

und

$R^{22}$ für eine typische Abgangsgruppe, wie beispielsweise Brom, Jod, Methan-, Toluol-, Fluor- oder Trifluormethansulfonyloxy, vorzugsweise für Brom steht,

mit Verbindungen der allgemeinen Formeln (III) oder (IIIa)

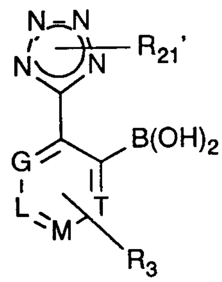

(III) oder

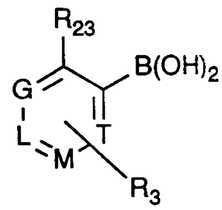

(IIIa)

in welcher

G, L, M, T und $R^3$ die oben angegebene Bedeutung haben

$R^{21'}$ für Wasserstoff oder für die Triphenylmethylgruppe steht,

und

$R^{23}$ die oben angegebene Bedeutung von $R^4$ hat, aber nicht für den Tetrazolylsubstituenten steht,

in inerten Lösemitteln, in Anwesenheit einer Base und metallkatalysiert umsetzt,

und anschließend im Fall $R^{21'}$ = Triphenylmethylgruppe diese Gruppe mit Säuren in organischen Lösemitteln und/oder Wasser nach üblichen Bedingungen abspaltet,

und im Fall der Salze bevorzugt ausgehend vom freien Tetrazol ($R^{21}/R^{21'}$ = H) mit Säuren oder Basen umsetzt,

und im Fall der freien Säure $R^4$ = $CO_2H$ und des freien Tetrazols $R^{21}/R^{21'}$ = H, ausgehend von den Salzen mit Säuren umsetzt,

und gegebenenfalls im Fall der unter den Substituenten $R^4$ aufgeführten carboxylischen Reste nach Verseifung der jeweiligen Ester beispielsweise durch Amidierung oder Sulfoamidierung nach üblichen Methoden derivatisiert,

und gegebenenfalls die Substituenten nach bekannten Methoden auf jeder Verfahrensstufe variiert.

Das erfindungsemäße Verfahren kann durch folgendes Formelschema beispielhaft erläutert werden:

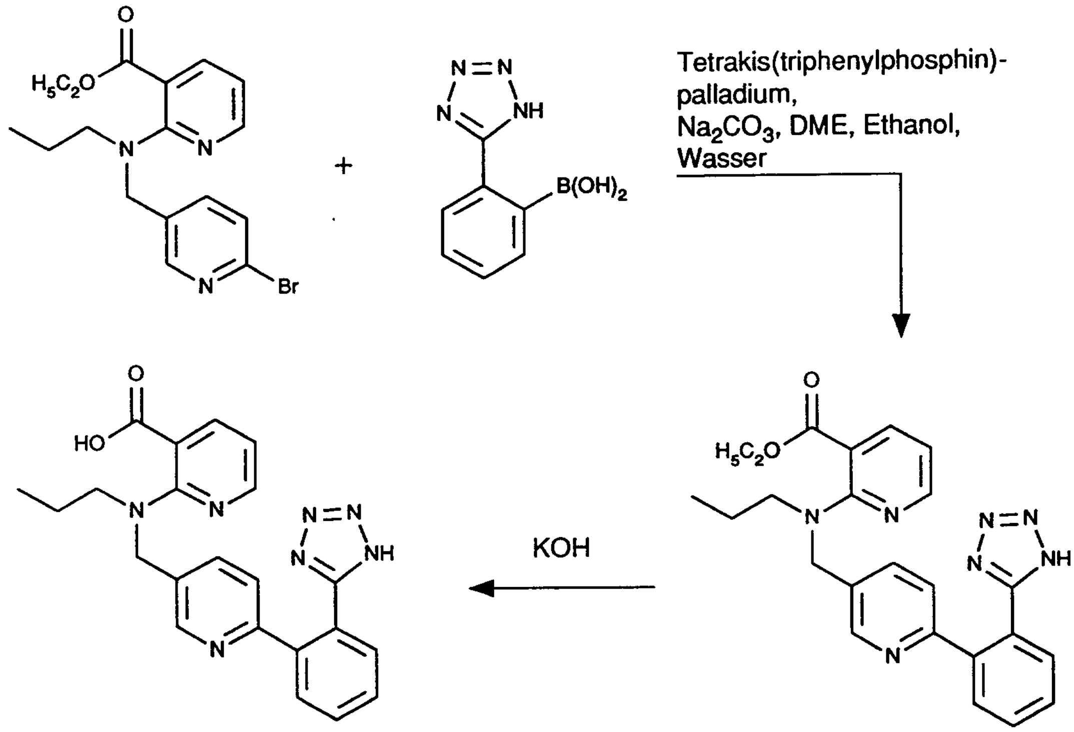


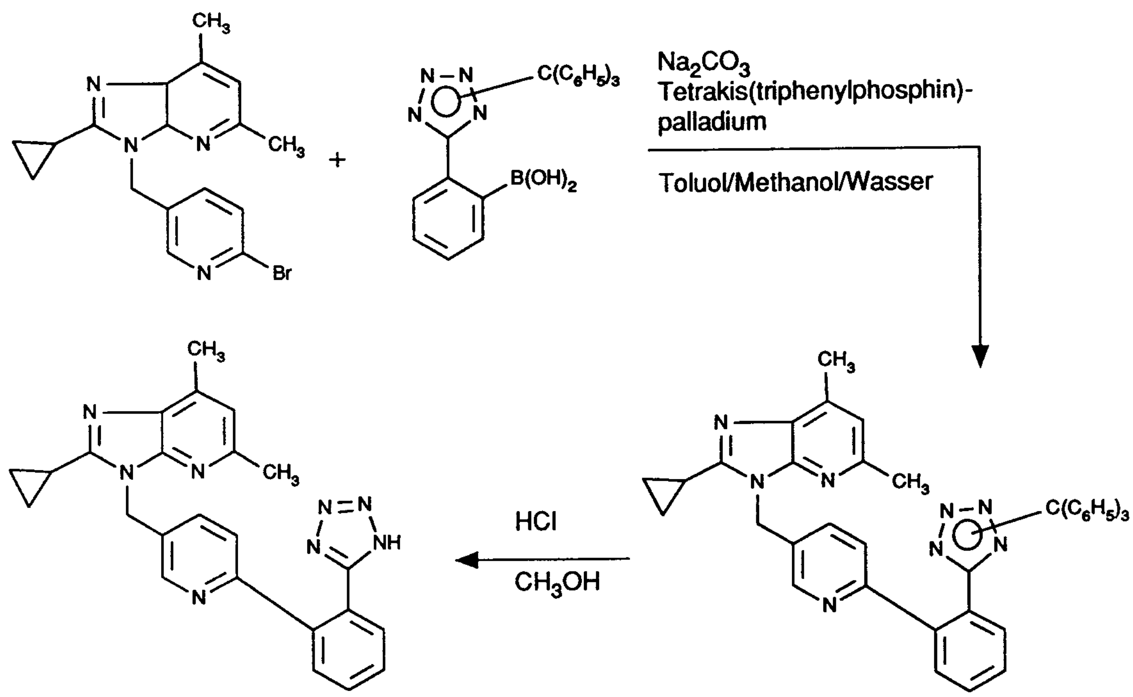


Als Lösemittel für das Verfahren eignen sich übliche organische Lösemittel, die sich unter den Reaktionsbedingungen nicht verändern. Hierzu gehören bevorzugt Wasser oder Alkohole, wie beispielsweise Methanol, Ethanol und Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid oder Dimethoxyethan, Hexamethylphosphorsäuretriamid, Acetonitril, Aceton oder Nitromethan. Ebenso ist es möglich, Gemische der genannten Lösemittel

zu verwenden. Bevorzugt sind Tetranydrofuran, Aceton, Dimethylformamid, Dimethoxyethan, Toluol und Methanol/Wasser.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkalioder Erdalkalialkoholate oder -amide wie Natrium- oder Kaliummethanolat, Natrium- oder Kaliumethanolat oder Kalium-tert.butylat, Thalliumcarbonat- oder -hydroxid, oder Lithiumdiisopropylamid (LDA), oder organische Amine (Trialkyl($C_1$-$C_6$)amine) wie Triethylamin oder Diisopropylethylamin, oder Heterocyclen wie 1,4-Diazabicyclo[2.2.2]octan (DABCO), 1,8-Diazabicyclo[5.4.0]undec-7-en (DBU), Pyridin, Dimethylaminopyridin, Methylpiperidin oder Morpholin. Es ist auch möglich als Basen Alkalimetalle, wie Natrium oder deren Hydride wie Natriumhydrid einzusetzen. Bevorzugt sind Kaliumcarbonat, Natriumcarbonat, Natriumhydrid, Kalium-tert.-butylat, Caesiumcarbonat, Thalliumhydroxid oder -carbonat.

Im allgemeinen setzt man die Base in einer Menge von 0,05 mol bis 10 mol, bevorzugt von 1 mol bis 2 mol jeweils bezogen auf 1 mol der Verbindung der Formel (III) ein.

Die erfindungsgemäßen Verfahren werden im allgemeinen in einem Temperaturbereich von -100 °C bis +100 °C, bevorzugt von 0 °C bis 80 °C und Schutzgasatmosphäre durchgeführt.

Als Katalysatoren eignen sich im allgemeinen Metallkomplexe des Nickels, Palladiums oder Platins, bevorzugt Palladium(O)-Komplexe wie beispielsweise Tetrakistriphenylphosphinpalladium. Ebenso ist es möglich Phasen-Transfer-Katalysatoren, wie beispielsweise Tetra-n-butylammoniumbromid oder Kronenether einzusetzen.

Außerdem eignen sich als Katalysatoren Kalium- oder Natriumiodid, bevorzugt Natriumiodid.

Der Katalysator wird in einer Menge von 0,005 mol bis 0,2 mol, bevorzugt von 0,01 mol bis 0,05 mol, bezogen auf 1 mol der Verbindung der allgemeinen Formel (III) oder (IIIa) eingesetzt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen.

Die Abspaltung erfolgt im allgemeinen in einem Temperaturbereich von 0 °C bis 150 °C, vorzugsweise von 20 °C bis 100 °C und Normaldruck.

Die Alkylierung erfolgt im allgemeinen mit Alkylierungsmitteln wie beispielsweise ($C_1$-$C_{10}$)-Alkylhalogeniden, Sulfonsäurestern oder substituierten oder unsubstituierten ($C_1$-$C_{10}$)-Dialkyl- oder ($C_1$-$C_{10}$)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20 °C bis +80 °C, vorzugsweise von -10 °C bis +30 °C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die saurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel (II) sind größtenteils neu und können beispielsweise hergestellt werden, indem man im Fall, daß V für den Rest der Formel

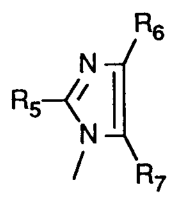

oder

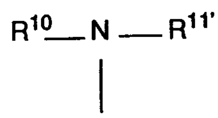

steht
worin

$R^5$, $R^6$, $R^7$ und $R^{10}$ die oben angegebene Bedeutung haben

und

$R^{11'}$ für das oben unter $R^{11}$ aufgeführte, gegebenenfalls substituierte Pyridin steht,

entweder Verbindungen der allgemeinen Formel (IV) oder (V)

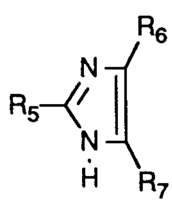

(IV)

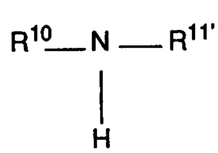

(V)

in welcher

$R^5$, $R^6$, $R^7$ und $R^{11'}$ die oben angegebene Bedeutung haben

mit Verbindungen der allgemeinen Formel (VI)

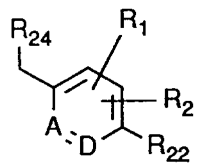

(VI)

in welcher

A, D, $R^1$, $R^2$ und $R^{22}$ die oben angegebene Bedeutung haben

und

$R^{24}$ für Halogen, vorzugsweise für Brom steht,

in einem der oben aufgeführten Lösemittel und der dort beschriebenen Basen, vorzugsweise Dimethylsulfoxid und Natriumhydrid umsetzt,

und im Fall, daß V für den Rest der Formel

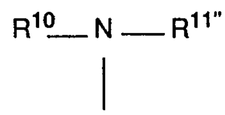

steht,

worin

$R^{10}$ die oben angegebene Bedeutung hat

und

$R^{11''}$ die oben angegebene Bedeutung von $R^{11}$ hat, aber nicht für gegebenenfalls substituiertes Pyridyl steht,

zunächst mit Verbindungen der allgemeinen Formel (VII)

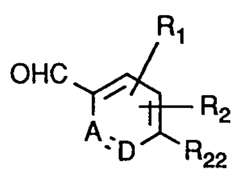

(VII)

in welcher

A, D, $R^1$, $R^2$ und $R^{22}$ die oben angegebene Bedeutung haben,

durch reduktive Aminierung nach bekannten Methoden mit Aminen der allgemeinen Formel (VIII)

$H_2N\text{-}R^{25}$ (VIII)

in welcher

$R^{25}$ den jeweiligen oben angegebenen Bedeutungsumfang von $R^{10}$ oder $R^{11''}$ umfaßt,

in einem der oben aufgeführten Lösemittel, gegebenenfalls in Anwesenheit einer Base,

in die Verbindungen der allgemeinen Formel (IX)

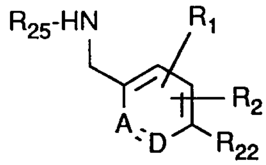

(IX)

in welcher
A, D, $R^1$, $R^2$, $R^{22}$ und $R^{25}$ die oben angegebene Bedeutung haben,
überführt,
und in einem letzten Schritt mit Isocyanaten der allgemeinen Formel(X)

$R^{26}$-N = C = O (X)

in welcher
$R^{26}$ in Abhängigkeit von dem oben aufgeführten Bedeutungsumfang des Substituenten $R^{25}$ entweder den oben angegebenen Bedeutungsumfang von $R^{10}$ und $R^{11''}$ umfaßt,
umsetzt.

Die erfindungsgemäßen Verfahren werden im allgemeinen in einem Temperaturbereich von -100 °C bis +100 °C, bevorzugt von 0 °C bis 80 °C und Schutzgasatmosphäre durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (IV), (V), (VI), (VII), (VIII) und (X) sind bekannt oder können dann nach bekannten Methoden hergestellt werden.

Die Verbindungen der allgemeinen Formel (IX) sind bekannt oder können dann beispielsweise wie oben beschrieben hergestellt werden.

Die Verbindungen der allgemeinen Formel (II), in welcher V für den Rest der Formel

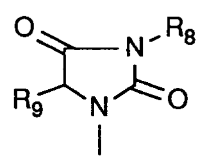

steht sind ebenfalls neu und können beispielsweise hergestellt werden, indem man zunächst Verbindungen der allgemeinen Formel (XI)

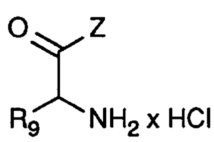

(XI)

in welcher
$R^9$ die oben angegebene Bedeutung hat
und
Z für $C_1$-$C_4$-Alkoxy steht,
durch Umsetzung mit Verbindungen der allgemeinen Formel (VI) wie oben beschrieben in die Verbindungen der allgemeinen Formel (XII)

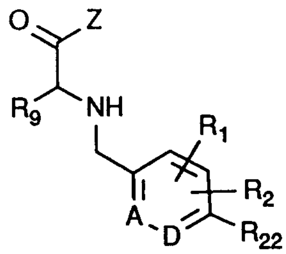

(XII)

in welcher

$R^1$, $R^2$, $R^9$, $R^{22}$, A, D und Z die oben angegebene Bedeutung haben,

überführt,

und in einem letzten Schritt mit Isocyanaten der allgemeinen Formel (XIII)

$R^8$-N=C=O (XIII)

in welcher

$R^8$ die oben angegebene Bedeutung hat,

in inerten Lösemitteln, in Anwesenheit einer Säure umsetzt.

Der erste Schritt des Verfahrens erfolgt im allgemeinen in einem der oben aufgeführten Lösemitteln und Basen, vorzugsweise Dimethylformamid oder Dichlormethan, Triethylamin und Kalium-tert.butylat in einem Temperaturbereich von 0 °C bis 100 °C, vorzugsweise bei Raumtemperatur und Normaldruck. Die Reaktion wird im allgemeinen unter Schutzgasatmosphäre durchgeführt.

Die Umsetzung mit den Isocyanaten erfolgt im allgemeinen in Essigester und Protonensäure, vorzugsweise mit Salzsäure in einem Temperaturbereich von 0 °C bis 100 °C, vorzugsweise von 60 °C bis 80 °C und Normaldruck.

Die Verbindungen der allgemeinen Formel (XI) sind bekannt oder nach bekannten Methoden herstellbar.

Ebenso sind die Verbindungen der allgemeinen Formel (XIII) bekannt.

Die Verbindungen der allgemeinen Formel (XII) sind neu und können wie oben beschrieben hergestellt werden.

Die Verbindungen der allgemeinen Formel (III) sind bekannt, im Fall $R^{21}$ = H neu und können dann hergestellt werden, indem man Phenyltetrazol zunächst unter Schutzgasatmosphäre, in einem aprotischen Lösemittel und in Anwesenheit einer Base umsetzt, anschließend Borsäuretrimethylester zufügt und in einem letzten Schritt mit Säuren hydrolysiert.

Als Lösemittel eignen sich für das Verfahren aprotische Lösemittel wie Ether, beispielweise Tetrahydrofuran, Diethylether, Toluol, Hexan oder Benzol. Bevorzugt ist Tetrahydrofuran.

Als Basen eignen sich prim-, sec.- und tert.Butyllithium und Phenyllithium. Bevorzugt ist n-Butyllithium.

Die Base wird in einer Menge von 2 mol bis 5 mol, bevorzugt von 2 mol bis 3 mol bezogen auf 1 mol Phenyltetrazol eingesetzt.

Als Säuren eignen sich im allgemeinen Mineralsäuren, wie beispielsweise Salzsäure, $C_1$-$C_4$-Carbonsäuren, wie beispielsweise Essigsäure oder Phosphorsäuren. Bevorzugt ist Salzsäure.

Die Säure wird im allgemeinen in einer Menge von 1 mol bis 10 mol, bevorzugt von 1 mol bis 3 mol, eingesetzt.

Das Verfahren wird im allgemeinen in einem Temperaturbereich von -70 °C bis +25 °C, bevorzugt bei -10 °C bis 0 °C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen substituierten Mono- und Bipyridylmethylderivate zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die

Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Außerdem können die Verbindungen zur Bekämpfung von Glaukom, diabetischer Retinopathie, Bewegungssteigerung der intraokularen Netzhautflüssigkeit eingesetzt werden.

Sie sind auch geeignet zur Bekämpfung von Erkrankungen des zentralen Nervensystems wie beispielsweise Depression, Migräne, Schizophrenie oder Angstzuständen, von Hirnleistungen, Schlaganfalll, diabetischer Nephropathie, Herzrhythmusstörungen, Prophylaxe von koronaren Herzerkrankungen oder Restenoseprophylaxe nach Angioplastien und gefäßchirugischen Maßnahmen.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogenbegaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen Applikationsvolumen pro Einzelgabe = 100 µl):

| | | |
|---|---|---|
| KCl | 22,7;32,7;42,7;52,7 | mmol/l |
| Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5

mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turtax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3$H-Angiotensin II (3-5 nM), Test-Pufferlösung (50 mM Tris, pH 7,2, 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt. Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel

als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen

Beispiel I

2-Cyclopropyl-5,7-dimethyl-3-(6-brompyridin-3-ylmethyl)imidazo[4,5-b]pyridin

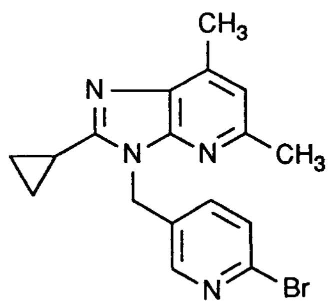

In 15 ml Dimethylsulfoxid werden 255 mg (6,4 mmol) Natriumhydrid und 994 mg (5,3 mmol) 2-Cyclopropyl-5,7-dimethy-imidazo[4,5-b]pyridin 2 h bei 0 °C gerührt. Nach Zugabe von 2 g (7,97 mmol) 2-Brom-5-brommethylpyridin wird über Nacht bei Raumtemperatur gerührt. Das Reaktionsgemisch wird mit Wasser verdünnt, zweimal mit Essigester gewaschen, die vereinigten organischen Phasen mit Wasser und Natriumchloridlösung gewaschen, über Natriumsulfat getrocknet und an 150 g Kieselgel mit Essigester/Petrolether-Gemischen 1:2 -> 1:0 zu 1,32 g der Titelverbindung chromatographiert.

Ausbeute: 69,7% der Theorie

$R_f$ = 0,45 (Kieselgel 60, Essigester)

Beispiel II

(S)-N-(1-Ethoxycarbonyl-2-methyl-prop-1-yl)-N-(6-brompyridin-3-ylmethyl)-amin

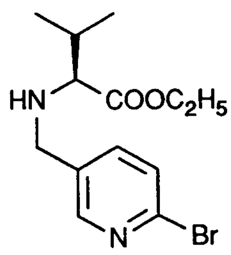

Eine Lösung von 4,77 g (25,65 mmol) 6-Brom-3-pyridin-aldehyd, 4,66 g (25,65 mmol) L-Valinethylesterhydrochlorid, 4,24 g (51,3 mmol) Natriumacetat in 400 ml Methanol wird 30 min mit 25 g 4 A Molekularsieb gerührt. Nach Zugabe von 3,22 g (51,3 mmol) Natriumcyanoborhydrid wird über Nacht bei Raumtemperatur gerührt, mit verdünnter Salzsäure auf pH = 2 eingestellt, mit Natriumcarbonat auf pH 10 eingestellt und über Kieselgur abgesaugt. Die Lösung wird mit Wasser verdünnt, dreimal mit Essigester gewaschen, die vereinigten organischen Phasen mit Natriumchloridlösung gewaschen, über Natriumsulfat getrocknet und an 350 g Kieselgel mit Essigester/Toluol-Gemischen (1:20 -> 1:10) zu 1,98 g der Titelverbindung chromatographiert

Ausbeute: 24% der Theorie

$R_f$ = 0,1 (Kieselgel 60, Essigester / Toluol 1:20)

Beispiel III

(S)-N-(1-Ethoxycarbonyl-2-methyl-prop-1-yl)-N-(3-oxo-2-propyl-heptanoyl)-N-(6-brompyridin-3-ylmethyl)-amin

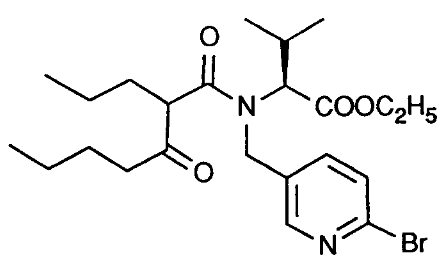

In 10 ml Dichlormethan werden 1,93 g (6,12 mmol) der Verbindung aus Beispiel II, 1,11 ml (9,2 mmol) Valeriansäurechlorid, 1,69 ml (12,2 mmol) Triethylamin und eine Spatelspitze 4-Dimethylaminopyridin bei 0 °C gerührt. Nach 18 h werden erneut 1,69 ml Triethylamin und 1,11 ml Valeriansäurechlorid zugegeben und über Nacht bei Raumtemperatur gerührt. Das Reaktionsgemisch wird mit Wasser verdünnt, mit Dichlormethan gewaschen, die organischen Phasen mit Wasser gewaschen, über Natriumsulfat getrocknet und an 150 g Kieselgel mit Essigester/Toluol 1:10 zu 1,13 g der Titelverbindung chromatographiert.

Ausbeute: 45.5% der Theorie

$R_f$ = 0,31 (Kieselgel 60, Essigester/Toluol 1:10)

Beispiel IV

(S)-N-(6-Brompyridin-3-ylmethyl)-N-(1-methoxycarbonylbut-1-yl)amin

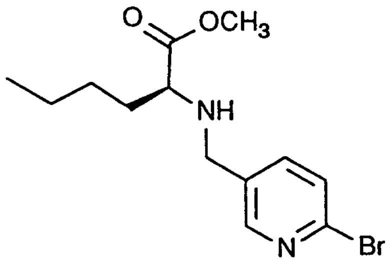

Eine Lösung von 2,18 g (12 mmol) (S)-2-Aminohexansäuremethylester-Hydrochlorid und 1,66 ml Triethylamin in 20 ml DME und 15 ml DMF werden unter Argon mit 1,48 g (13,2 mmol) Kalium-tert.butylat versetzt. Nach 1 h Rühren bei 20 °C wird eine Lösung von 3,01 g (12 mmol) 2-Brom-5-brommethylpyridin in 15 ml DMF injiziert und 16 h gerührt Anschließend wird das Solvens im Vakuum abdestilliert, der Rückstand mit Dichlormethan/Wasser aufgenommen, die organische Phase über Natriumsulfat getrocknet und nach Einengen an Kieselgel mit Petrolether/Essigester (4:1) gereinigt.
Ausbeute: 1,64 g (46% der Theorie)
$R_f$ = 0,32 (Petrolether/Essigester = 3:1)

Beispiel V

(S)-1-(6-Brompyridin-3-ylmethyl)-3,5-dibutyl-imidazolidin-2,4-dion

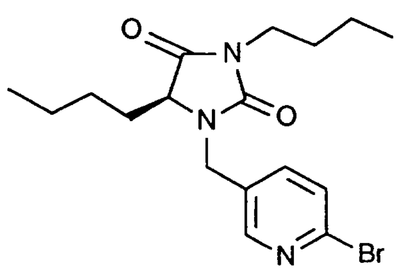

Eine Lösung von 900 mg (2,84 mmol) der Verbindung aus Beispiel IV und 845 mg (8,52 mmol) Butylisocyanat in 20 ml Essigester werden 16 h am Rückfluß erhitzt. Anschließend werden 20 ml 6 M Salzsäure zugegeben, 1 h bei 60 °C gerührt, mit 30 ml Essigester verdünnt und mit 20 ml Wasser gewaschen. Die organische Phase wird abgetrennt, über Natriumsulfat getrocknet und zu 1,1 g Rohprodukt eingeengt, das ohne Reinigung weiter umgesetzt wird.
$R_f$ = 0,66 (Toluol/Essigester/Eisessig = 10:30:1)

Beispiel VI

N-(6-Brompyridin-3-ylmethyl)-N-butylamin

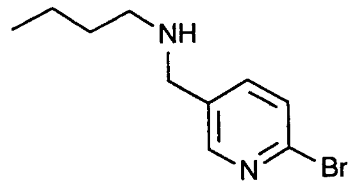

Eine Lösung von 2 g (7,97 mmol) 2-Brom-5-brommethylpyridin und 2,91 g (39,85 mmol) n-Butylamin in 20 ml Dioxan wird 2 h unter Rückfluß erhitzt. Anschließend wird das Solvens im Vakuum entfernt, mit Essigester/Dichlormethan aufgenommen und mit Wasser und ges. Natriumchlorid-Lösung gewachen. Die organische Phase wird über Natriumsulfat getrocknet, eingeengt und der Rückstand an Kieselgel mit Dichlormethan/Methanol (95:5) gereinigt.
Ausbeute: 1,42 g (73% der Theorie)
$R_f$ = 0,49 (Dichlormethan/Methanol = 95:5)

Beispiel VII

(S)-N-(6-Brompyridin-3-ylmethyl)-N-butyl-N'-(1-methoxycarbonyl-2-methylbutyl)-harnstoff

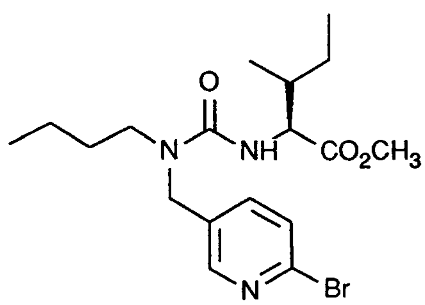

Zu einer Lösung von 0,93 g (2,16 mmol) (S)-Isoleucinmethylesterisocyanat (hergestellt durch Umsetzung von (S)-Isoleucinmethylester-Hydrochlorid mit Chlorameisensäure-trichlormethylester in Toluol bei 120 °C) in 10 ml Essigester wird bei 0 °C eine Lösung von 0,73 g (3 mmol) der Verbindung aus Beispiel VI in 5 ml Essigester gegeben und 30 min bei dieser Temperatur gerührt. Anschließend wird die Reaktionsmischung nacheinander mit verdünnter Salzsäure, Wasser, ges. Natriumhydrogencarbonat- und ges. Natriumchlorid-Lösung gewaschen, die organische Phase eingeengt und der Rückstand an Kieselgel mit Petrolether/Essigester (2:1) gereinigt.
Ausbeute: 658 mg (74% der Theorie)
$R_f$ = 0,53 (Petrolether/Essigester = 1:1)

Beispiel VIII

2-{[N-(6-Brompyridin-3-ylmethyl)-N-propyl]amino}pyridin-3-carbonsäureethylester

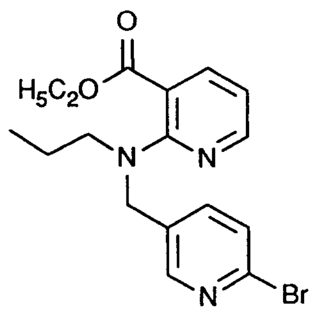

Zu einer Lösung von 0,87 g (5,4 mmol) Hexamethyldisilazan in 5,5 ml THF werden bei -20 °C 3,2 ml einer 1,6 N Lösung von n-Butyllithium gegeben. Die so zubereitete Lösung wird bei -10 °C bis 0 °C zu einer Lösung von 1,07 g (5,13 mmol) 2-Propylaminopyridin-3-carbonsäureethylester und 1,25 g 1,3-Dimethyltetrahydro-2(1H)-pyrimidinon (DMPU) in 4 ml THF getropft. Nach 10 min Rühren bei dieser Temperatur werden 2 g (8 mmol) 2-Brom-5-brommethylpyridin gelöst in 12 ml THF langsam zugetropft. Nach Entfernen des Kühlbades wird 36 h bei 20 °C gerührt. Danach werden 2 Tropfen konz. Salzsäure zugegeben, das Solvens im Vakuum entfernt, der Rückstand in Essigester aufgenommen und dreimal mit Wasser gewaschen. Die organische Phase wird über Natriumsulfat getrocknet, eingeengt und der Rückstand an Kieselgel mit Petrolether/Essigester (20:1) gereinigt.
Ausbeute: 1,14 g (59% der Theorie)
$R_f$ = 0,35 (Petrolether/Essigester = 5:1)

Herstellungsbeispiele

Beispiel 1

2-Cyclopropyl-5,7-dimethyl-3-{6-[2-(1-triphenylmethyl-tetrazol-5-yl)-phenyl]-pyridin-3-yl-methyl}imidazo-[4,5-b]pyridin

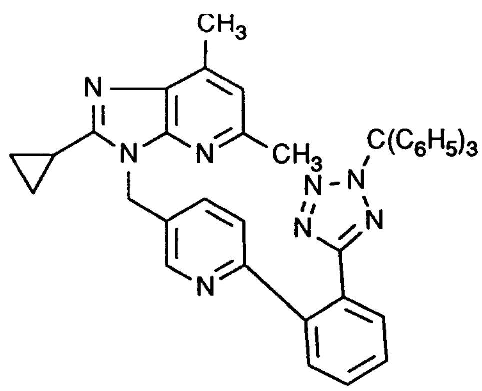

Eine Suspension aus 1,3 g (3,64 mmol) der Verbindung aus Beispiel I,1,73 g (4,0 mmol) 3-(2'-Triphenylmethyl-2'H-tetrazol-5'-yl)phenylboronsäure, 772 mg (7,28 mmol) Natriumcarbonat, 420 mg (0,36 mmol) Tetrakis(triphenylphosphin)palladium, 4 ml Methanol, 4 ml Wasser und 40 ml Toluol werden unter Argonatmosphäre über Nacht unter Rückfluß erhitzt. Das Reaktionsgemisch wird mit Wasser und Natriumchloridlösung gewaschen, über Natriumsulfat getrocknet und an 200 g Kieselgel mit Essigester/Petrolether (1:1) zu 1,53 g der Titelverbindung chromatographiert.

Ausbeute: 63,2% der Theorie
$R_f$ = 0,11 (Kieselgel 60, Essigester/Petrolether 1:1)

Beispiel 2

2-Cyclopropyl-5,7-dimethyl-3-{6-[2-(1H-tetrazol-5-yl)phenyl]-pyridin-3-yl-methyl}-imidazo[4,5-b]pyridin

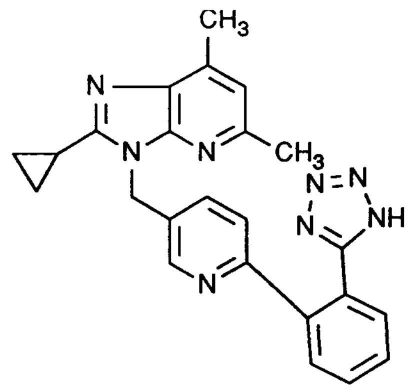

In 15 ml Methanol werden 0,75 g (1,13 mmol) der Verbindung aus Beispiel 1 und 0,1 ml konzentrierter Salzsäure über Nacht bei Raumtemperatur gerührt. Das Reaktionsgemisch wird mit Wasser verdünnt, mit Essigester dreimal gewaschen, die vereinigten organischen Phasen mit Wasser und Natriumchloridlösung gewaschen, über Natriumsulfat getrocknet und an 100 g Kieselgel mit Dichlormethan/Methanol-Gemischen (20:1 -> 0:1) zu 175 mg der Titelverbindung chromatographiert.
Ausbeute: 36,3 g der Theorie
$R_f$ = 0,50 (Kieselgel 60, Acetonitril/Wasser 5:1)
MS (FAB): 423 (M+H), 445 (M+Na)
In Analogie zur den Vorschriften der Beispiele 1 und 2 werden die in Tabelle 1 aufgeführten Verbindungen erhalten.

Tabelle 1:

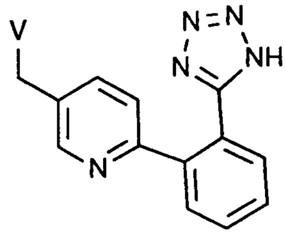

| Bsp.-Nr. | V | MS (FAB) |
| --- | --- | --- |
| 3 | 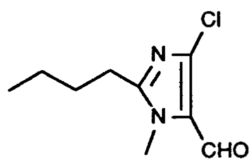 | 422/424 (M+1)<br>444/446 (M+Na) |
| 4 | 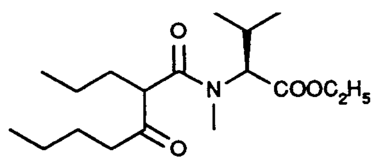 | 549 (M+1) |
| 5 | 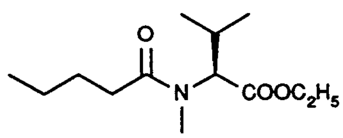 | 465 (M+1) |

Beispiel 6

(S)-3,5-Dibutyl-1-{6-[2-(1H-tetrazol-5-yl)phenyl]pyridin-3-ylmethyl}-imidazolidin-2,4-dion

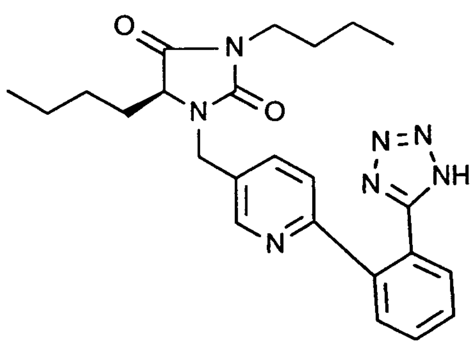

Eine Lösung von 1,1 g (2,36 mmol) der Verbindung aus Beispiel V in 20 ml DME wird sukzessive mit 272 mg Tetrakistriphenylphosphinpalladium(NaH), 7,1 ml 2 M Natriumcarbonatlösung, 538 mg (2,83 mmol) 2-(Tetrazol-5'-yl)phenylboronsäure und 1 ml Ethanol versetzt und 16 h unter Rückfluß erhitzt. Nach Abkühlen wird die Reaktionsmischung über Kieselgur abgesaugt, mit Methanol nachgewaschen, das Solvens entfernt und an Kieselgel mit Toluol/Essigester/Eisessig (35:5:1) gereingt.
Ausbeute: 917 mg (87% der Theorie)

$R_f$ = 0,22 (Toluol/Essigester/Eisessig = 20:20:1)

Beispiel 7

(S)-N-Butyl-N-{6-[2-(1H-tetrazol-5-yl)phenyl]pyridin-3-ylmethyl}-N'-(1-methoxycarbonyl-2-methylbutyl)-harnstoff

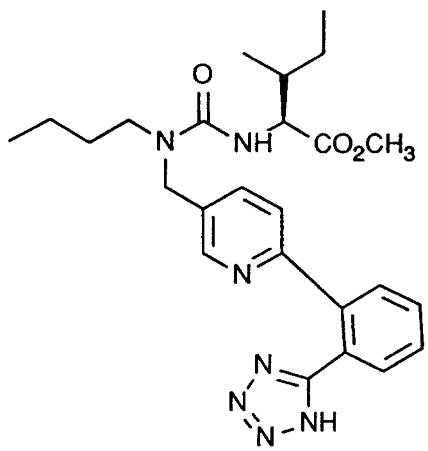

In Analogie zur Vorschrift des Beispiels 6 wird die Titelverbindung aus 658 mg (1,59 mmol) der Verbindung aus Beispiel VII und 363 mg (1,9 mmol) 2-(Tetrazol-5'-yl)phenylboronsäure erhalten.
Ausbeute: 123 mg (16% der Theorie)
$R_f$ = 0,52 (Toluol/Essigester/Eisessig = 20:20:1)

Beispiel 8

(S)-N-(1-Carboxy-2-methyl-prop-1-yl)-N-3-oxo-2-propyl-heptanoyl-N-{6-[2-(1H-tetrazol-5-yl)phenyl]pyridin-3-ylmethyl}amin

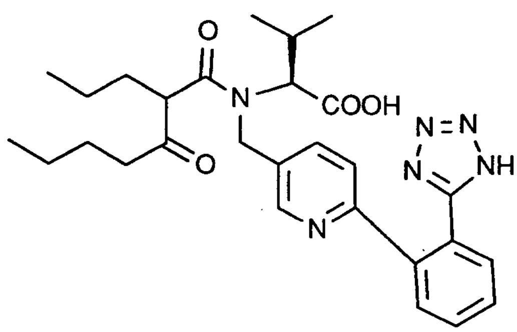

Eine Lösung aus 50 mg (0,9 mmol) der Verbindung aus Beispiel 4 in Ethanol und 2,3 ml eine 0,1 N Kaliumhydroxidlösung wird über Nacht bei 20 °C gerührt. Es wird mit verdünnter Salzsäure angesäuert, mit Essigester gewaschen, die vereinigten organischen Phasen über Natriumsulfat getrocknet und eingeengt.
Ausbeute: 73% der Theorie
$R_f$ (Acetonitril/Wasser, Kieselgel 10:1) = 0,36

Beispiel 9

(S)-N-Butyl-N-{6-[2-(1H-tetrazol-5-yl)phenyl]pyridin-3-yl-methyl}-N'-(1-carboxy-2-methylbutyl)harnstoff

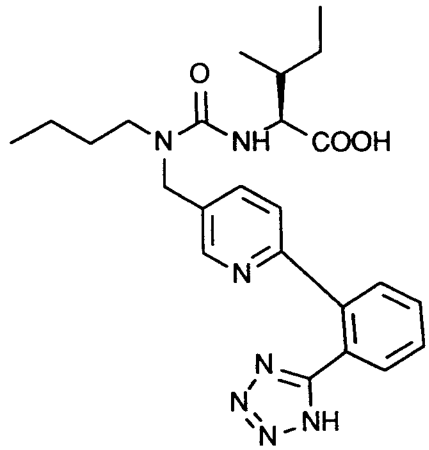

In Analogie zur Vorschrift des Beispiels 8 wird die Titelverbindung aus 110 mg (0,23 mmol) der Verbindung aus Beispiel 7 erhalten.
Ausbeute: 43 mg (40% der Theorie)
$R_f$ = 0,18 (Toluol/Essigester/Eisessig = 20:20:1)

Beispiel 10

2-{N-Propyl-N-[6-[2-(1H-tetrazol-5-yl)phenyl]pyridin-3-ylmethyl]amino}-pyridin-3-carbonsäureethylester

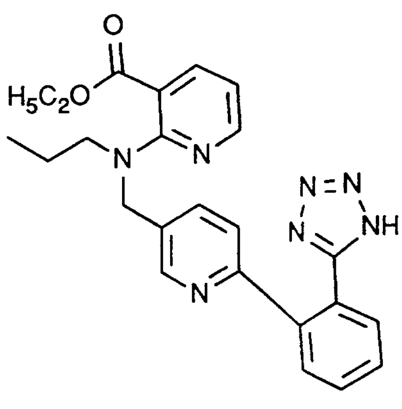

In Analogie zur Vorschrift des Beispiels 6 wird die Titelverbindung aus 1,14 g (3 mmol) der Verbindung aus Beispiel VIII und 0,69 g (3,61 mmol) 2-(Tetrazol-5'-yl)-phenylboronsäure erhalten.
Ausbeute: 807 mg (60% der Theorie)
$R_f$ = 0,47 (Toluol/Essigester/Eisessig = 20:20:1)

Beispiel 11

2-(N-Propyl-N-{6-[2-(1H-tetrazol-5-yl)phenyl]pyridin-3-yl-methyl]amino}pyridin-3-carbonsäure

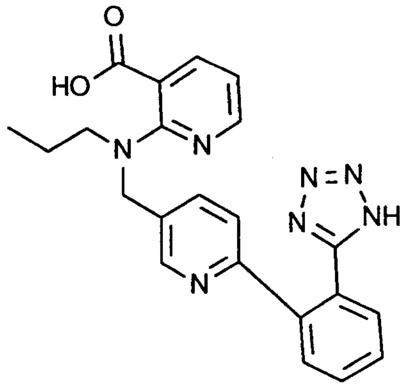

In Analogie zur Vorschrift des Beispiels 8 wird die Titelverbindung aus 320 mg (0,72 mmol) der Verbindung aus Beispiel 10 erhalten.
Ausbeute: 91 mg (31% der Theorie)
$R_f$ = 0,18 (Toluol/Essigester/Eisessig = 20:20:1)

Beispiel 12

2-Cyclopropyl-5,7-dimethyl-3-{6-[2-[1H-tetrazol-5-yl)phenyl]-pyridin-3-yl-methyl}-imidazo[4,5-b]pyridin-Kaliumsalz

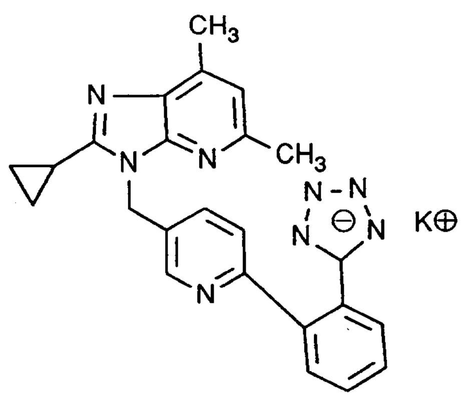

In 6 ml Methanol, 0,6 ml Wasser und 3 ml Tetrahydrofuran werden 129,4 ml (0,31 mmol) der Verbindung aus Beispiel 2 und 30,6 mg (0,31 mol) Kaliumhydrogencarbonat 2h bei Raumtemperatur gerührt. Es wird im Vakuum eingeengt, der Rückstand lyophilisiert und im Vakuum über Phosphorpentoxid zu 134 mg der Titelverbindung getrocknet.
Ausbeute: 95,1% der Theorie
MS (FAB): 423 (M + H), 445 (M + Na), 461 (M + K)
In Analogie zur Vorschrift des Beispiels 12 werden die in Tabelle 2 aufgeführten Verbindungen erhalten.

Tabelle 2:

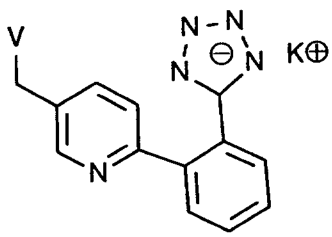

| Bsp.-Nr. | V | MS (FAB) |
|---|---|---|
| 13 | 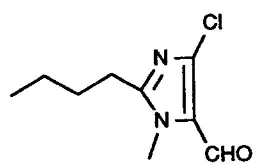 | 422/424 (M+H)<br>444/446 (M+Na)<br>460/462 (M+K) |
| 14 | 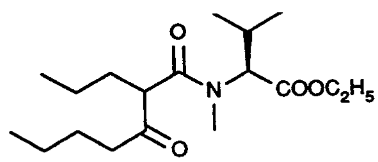 | 549 (M+H)<br>587 (M+K) |
| 15 | 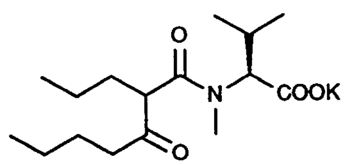 | |
| 16 | 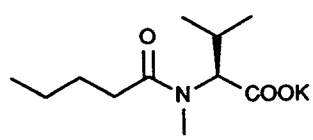 | |

**Patentansprüche**

**1.** Substituierte Mono- und Bipyridylmethylderivate der allgemeinen Formel (I),

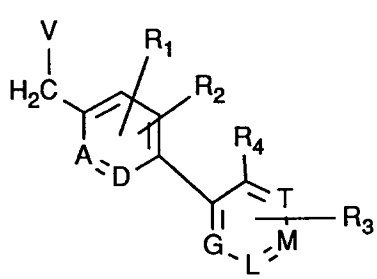

(I)

in welcher

A, D, G, L, M und T gleich oder verschieden sind und für die CH-Gruppe oder für ein Stickstoffatom stehen, wobei aber mindestens einer dieser Reste und maximal jeweils

einer dieser Reste in jedem Cyclus für ein Stickstoffatom stehen darf,

V für einen Rest der Formel

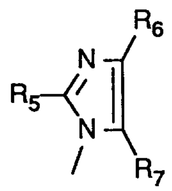
,
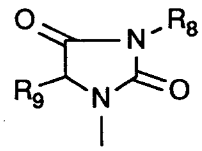
oder
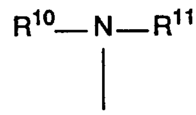

steht,
worin

$R^5$, $R^8$ und $R^9$ gleich oder verschieden sind und geradkettiges oder verzweigtes Alkyl mit bis zu 10 Kohlenstoffatomen bedeuten, das gegebenenfalls durch Cycloalkyl mit 3 bis 6 Kohlenstoffatomen, Hydroxy oder durch geradkettiges oder verzweigtes Alkoxy oder Alkylthio mit jeweils bis zu 6 Kohlenstoffatomen substituiert ist, oder Cycloalkyl mit 3 bis 6 Kohlenstoffatomen bedeuten,

$R^6$ Halogen bedeutet,

$R^7$ Formyl, Carboxy, geradkettiges oder verzweigtes Alkoxycarbonyl mit bis zu 6 Kohlenstoffatomen oder geradkettiges oder verzweigtes Alkyl mit bis zu 6 Kohlenstoffatomen bedeutet, das gegebenenfalls durch Hydroxy substituiert ist,

oder

$R^6$ und $R^7$ unter Einbezug der Doppelbindung gemeinsam einen Pyridylring bilden, der 2- bis 3-fach gleich oder verschieden durch Carboxy, geradkettiges oder verzweigtes Alkoxycarbonyl mit bis zu 6 Kohlenstoffatomen oder durch geradkettiges oder verzweigtes Alkyl mit bis zu 6 Kohlenstoffatomen substituiert ist, das seinerseits durch Hydroxy substituiert sein kann,

$R^{10}$ geradkettiges oder verzweigtes Alkyl mit bis zu 8 Kohlenstoffatomen, Cycloalkyl mit 3 bis 6 Kohlenstoffatomen oder geradkettiges oder verzweigtes Acyl mit bis zu 8 Kohlenstoffatomen bedeutet, das gegebenenfalls durch geradkettiges oder verzweigtes Acyl mit bis zu 8 Kohlenstoffatomen substituiert ist,

$R^{11}$ Pyridyl bedeutet, das gegebenenfalls bis zu 2-fach gleich oder verschieden durch Carboxy, geradkettiges oder verzweigtes Alkoxycarbonyl mit bis zu 6 Kohlenstoffatomen oder durch geradkettiges oder verzweigtes Alkyl mit bis zu 6 Kohlenstoffatomen substituiert ist, das seinerseits durch Hydroxy substituiert sein kann, oder

geradkettiges oder verzweigtes Alkyl mit bis zu 8 Kohlenstoffatomen bedeutet, das gegebenenfalls durch Carboxy oder durch geradkettiges oder verzweigtes Alkoxycarbonyl mit bis zu 6 Kohlenstoffatomen substituiert ist,

oder

eine Gruppe der Formel $-CO-NR^{12}R^{13}$ bedeutet,

worin

$R^{12}$ Wasserstoff oder Methyl bedeutet,

$R^{13}$ geradkettiges oder verzweigtes Alkyl mit bis zu 8 Kohlenstoffatomen bedeutet, das durch Carboxy oder geradkettiges oder verzweigtes Alkoxycarbonyl mit bis zu 6 Kohlenstoffatomen substituiert ist,

$R^1$, $R^2$ und $R^3$ gleich oder verschieden sind und für Wasserstoff, Halogen, Cyano, Nitro, Trifluormethyl, Amido oder für geradkettiges oder verzweigtes Alkyl oder Alkoxycarbonyl mit bis zu 6 Kohlenstoffatomen stehen,

$R^4$ für eine Gruppe der Formel $-CO-R^{14}$, $-SO_2R^{15}$, $-CO-NR^{16}R^{17}$, $-NH-SO_2R^{18}$ oder $-SO_2NR^{19}R^{20}$ steht,

worin

$R^{14}$ Hydroxy oder geradkettiges oder verzweigtes Alkoxy mit bis zu 6 Kohlenstoffatomen bedeutet,

$R^{15}$ Hydroxy, Trifluormethyl, geradkettiges oder verzweigtes Alkoxy oder Alkyl mit jeweils bis zu 6 Kohlenstoffatomen, Phenyl oder Benzyl bedeutet, die gegebenenfalls bis zu 2-fach gleich oder verschieden durch Halogen, Trifluormethyl oder geradkettiges oder verzweigtes Alkyl mit bis zu 4 Kohlenstoffatomen substituiert sind,

$R^{16}$ und $R^{17}$ gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,

oder

$R^{16}$ Wasserstoff bedeutet

und

$R^{17}$ die Gruppe $-SO_2R^{15}$ bedeutet,

worin

$R^{15}$ die oben angegebene Bedeutung hat,

$R^{18}$ die oben angegebene Bedeutung von $R^{15}$ hat und mit dieser gleich oder verschieden ist,

$R^{19}$ und $R^{20}$ die oben angegebene Bedeutung von $R^{16}$ und $R^{17}$ haben und mit diesen gleich oder verschieden sind,

oder

$R^{19}$ Wasserstoff oder geradkettiges oder verzweigtes Alkyl mit bis zu 4 Kohlenstoffatomen bedeutet

und

$R^{20}$ die oben angegebene Bedeutung von $R^{15}$ hat und mit dieser gleich oder verschieden ist,

oder

$R^4$ für einen Rest der Formel

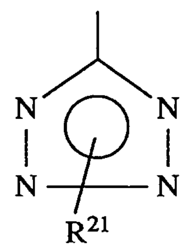

steht,

worin

$R^{21}$ Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,

und deren Salze.

**2.** Substituierte Mono- und Bipyridylmethylderivate nach Anspruch 1 worin

A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,

V für einen Rest der Formel

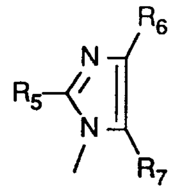 , 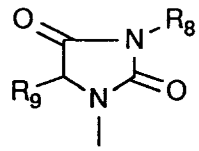 oder 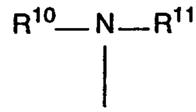

steht,

worin

$R^5$, $R^8$ und $R^9$ gleich oder verschieden sind und geradkettiges oder verzweigtes Alkyl mit jeweils bis zu 8 Kohlenstoffatomen bedeuten, das gegebenerfalls durch Cyclopropyl, Cyclopentyl, Cyclohexyl, Hydroxy oder durch geradkettiges oder verzweigtes Alkoxy oder Alkylthio mit jeweils bis zu 4 Kohlenstoffatomen substituiert ist, oder Cyclopropyl, Cyclopentyl oder Cyclohexyl bedeuten,

$R^6$ Fluor, Chlor oder Brom bedeutet,

$R^7$ Formyl, Carboxy, geradkettiges oder verzweigtes Alkoxycarbonyl mit bis zu 4 Kohlenstoffatomen oder geradkettiges oder verzweigtes Alkyl mit bis zu 4 Kohlenstoffatomen bedeutet, das gegebenenfalls durch Hydroxy substituiert ist,

oder

$R^6$ und $R^7$ unter Einbezug der Doppelbindung gemeinsam einen Pyridylring bilden, der 2- bis 3-fach gleich oder verschieden durch Carboxy, geradkettiges oder verzweigtes Alkoxycarbonyl mit bis zu 4 Kohlenstoffatomen oder durch geradkettiges oder verzweigtes Alkyl mit bis zu 4 Kohlenstoffatomen substituiert ist, das seinerseits durch Hydroxy substituiert sein kann,

$R^{10}$ geradkettiges oder verzweigtes Alkyl mit bis zu 6 Kohlenstoffatomen, Cyclopropyl, Cyclopentyl, Cyclohexyl oder geradkettiges oder verzweigtes Acyl mit bis zu 6 Kohlenstoffatomen bedeutet, das gegebenenfalls durch geradkettiges oder verzweigtes Acyl mit bis zu 6 Kohlenstoffatomen substituiert ist,

$R^{11}$ Pyridyl bedeutet, das gegebenenfalls bis zu 2-fach gleich oder verschieden durch Carboxy, geradkettiges oder verzweigtes Alkoxycarbonyl mit bis zu 4 Kohlenstoffatomen oder durch geradkettiges oder verzweigtes Alkyl mit bis zu 4 Kohlenstoffatomen substituiert ist, das seinerseits durch Hydroxy substituiert sein kann, oder geradkettiges oder verzweigtes Alkyl mit bis zu 6 Kohlenstoffatomen bedeutet, das gegebenenfalls durch Carboxy oder durch geradkettiges oder verzweigtes Alkoxycarbonyl mit bis zu 4 Kohlenstoffatomen substituiert ist,oder eine Gruppe der Formel $-CO-NR^{12}R^{13}$ bedeutet,

worin

$R^{12}$ Wasserstoff oder Methyl bedeutet,

$R^{13}$ geradkettiges oder verzweigtes Alkyl mit bis zu 6 Kohlenstoffatomen bedeutet, das durch Carboxy oder geradkettiges oder verzweigtes Alkoxycarbonyl mit bis zu 4 Kohlenstoffatomen substituiert ist,

$R^1$, $R^2$ und $R^3$ gleich oder verschieden sind und für Wasserstoff, Fluor, Chlor, Brom, Trifluormethyl, Amido oder für geradkettiges oder verzweigtes Alkyl oder Alkoxycarbonyl mit jeweils bis zu 4 Kohlenstoffatomen stehen,

$R^4$ für eine Gruppe der Formel $-CO-R^{14}$, $-SO_2R^{15}$, $-CO-NR^{16}R^{17}$, $-NH-SO_2R^{18}$ oder $-SO_2NR^{19}R^{20}$ steht,

worin

$R^{14}$ Hydroxy oder geradkettiges oder verzweigtes Alkoxy mit bis zu 4 Kohlenstoffatomen bedeutet,

$R^{15}$ geradkettiges oder verzweigtes Alkyl mit bis zu 4 Kohlenstoffatomen, Benzyl, Trifluormethyl oder p-Tolyl bedeutet,

$R^{16}$ und $R^{17}$ gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,

oder

$R^{16}$ Wasserstoff bedeutet

und

$R^{17}$ die Gruppe $-SO_2R^{15}$ bedeutet,
worin

$R^{15}$ die oben angegebene Bedeutung hat,

$R^{18}$ die oben angegebene Bedeutung von $R^{15}$ hat und mit dieser gleich oder verschieden ist,

$R^{19}$ und $R^{20}$ die oben angegebene Bedeutung von $R^{16}$ und $R^{17}$ haben und mit dieser gleich oder verschieden sind,
oder

$R^{19}$ Wasserstoff oder Methyl bedeutet,

$R^{20}$ die oben angegebene Bedeutung von $R^{15}$ hat und mit dieser gleich oder verschieden ist,
oder

$R^4$ für einen Rest der Formel

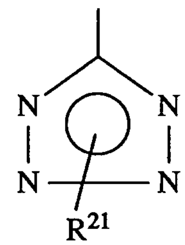

steht,
worin

$R^{21}$ Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,

und deren Salze.

**3.** Substituierte Mono- und Bipyridylmethylderivate nach Anspruch 1
wobei

A, D, G, L, M und T gleich oder verschieden sind und für die CH-Gruppe oder für ein Stickstoffatom stehen, wobei aber mindestens einer der Reste und maximal jeweils einer der Reste in jedem Cyclus für ein Stickstoffatom stehen darf,

V für einen Rest der Formel

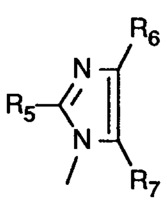

,

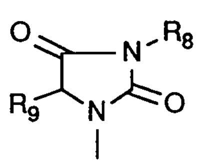

oder

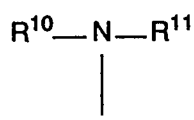

s teht,
worin

$R^5$, $R^8$ und $R^9$ gleich oder verschieden sind und geradkettiges oder verzweigtes Alkyl mit bis zu 6 Kohlenstoffatomen bedeuten, das gegebenenfalls durch Cyclopropyl, Hydroxy, geradkettiges oder verzweigtes Alkoxy oder Alkylthio mit jeweils bis zu 3 Kohlenstoffatomen substituiert ist oder
Cyclopropyl, Chlor oder Iod bedeuten,

$R^6$ Fluor, Chlor oder Brom bedeutet,

$R^7$ Formyl, Carboxy, geradkettiges oder verzweigtes Alkoxycarbonyl mit bis zu 3 Kohlenstoffatomen oder geradkettiges oder verzweigtes Alkyl mit bis zu 4

Kohlenstoffatomen bedeutet, das gegebenenfalls durch Hydroxy substituiert ist,
oder

$R^6$ und $R^7$ unter Einbezug der Doppelbindung gemeinsam einen Pyridylring bilden, der 2- oder 3-fach gleich oder verschieden durch Carboxy, geradkettiges oder verzweigtes Alkoxycarbonyl mit bis zu 3 Kohlenstoffatomen oder durch geradkettiges oder verzweigtes Alkyl mit bis zu 3 Kohlenstoffatomen substituiert ist, das seinerseits durch Hydroxy substituiert sein kann,

$R^{10}$ geradkettiges oder verzweigtes Alkyl mit bis zu 5 Kohlenstoffatomen, Cyclopropyl, Cyclopentyl, Cyclohexyl oder geradkettiges oder verzweigtes Acyl mit bis zu 5 Kohlenstoffatomen bedeutet, das gegebenenfalls durch geradkettiges oder verzweigtes Acyl mit bis zu 5 Kohlenstoffatomen substituiert ist,

$R^{11}$ Pyridyl bedeutet, das gegebenenfalls bis zu 2-fach gleich oder verschieden durch Carboxy, geradkettiges oder verzweigtes Alkoxycarbonyl mit bis zu 3 Kohlenstoffatomen oder durch geradkettiges oder verzweigtes Alkyl mit bis zu 3 Kohlenstoffatomen substituiert ist, das seinerseits durch Hydroxy substituiert sein kann, oder
geradkettiges oder verzweigtes Alkyl mit bis zu 5 Kohlenstoffatomen bedeutet, das gegebenenfalls durch Carboxy oder durch geradkettiges oder verzweigtes Alkoxycarbonyl mit bis zu 3 Kohlenstoffatomen substituiert ist,oder
eine Gruppe der Formel $-CO-NR^{12}R^{13}$ bedeutet,
worin

$R^{12}$ Wasserstoff oder Methyl bedeutet,

$R^{13}$ geradkettiges oder verzweigtes Alkyl mit bis zu 6 Kohlenstoffatomen bedeutet, das durch Carboxy oder geradkettiges oder verzweigtes Alkoxycarbonyl mit bis zu 3 Kohlenstoffatomen substituiert ist,

$R^1$, $R^2$ und $R^3$ gleich oder verschieden sind und
für Wasserstoff, Fluor, Chlor, Brom oder Methyl stehen,

$R^4$ für eine Gruppe der Formel $-CO-R^{14}$, $-SO_2-R^{15}$, $-CO-NR^{16}R^{17}$, $-NH-SO_2R^{18}$ oder $-SO_2-NR^{19}R^{20}$ bedeutet,
worin

$R^{14}$ Hydroxy oder geradkettiges oder verzweigtes Alkoxy mit bis zu 3 Kohlenstoffatomen bedeutet,

$R^{15}$ Methyl, Trifluormethyl, Benzyl oder p-Tolyl bedeutet,

$R^{16}$ und $R^{17}$ gleich oder verschieden sind und Wasserstoff, Cyclopropyl, Cyclopentyl oder geradkettiges oder verzweigtes Alkyl mit bis zu 4 Kohlenstoffatomen bedeuten, das gegebenenfalls durch Phenyl substituiert ist,

$R^{16}$ Wasserstoff bedeutet und

$R^{17}$ die Gruppe $-SO_2-R^{15}$ bedeutet,
worin

$R^{15}$ die oben angegebene Bedeutung hat,

$R^{18}$ die oben angegebene Bedeutung von $R^{15}$ hat und mit dieser gleich oder verschieden ist,

$R^{19}$ und $R^{20}$ die oben angegebene Bedeutung von $R^{16}$ und $R^{17}$ haben und mit dieser gleich oder verschieden sind,
oder

$R^{19}$ Wasserstoff oder Methyl bedeutet
und

$R^{20}$ die oben angegebene Bedeutung von $R^{15}$ hat und mit dieser gleich oder verschieden ist
oder

$R^4$ für den Tetrazolylrest der Formel

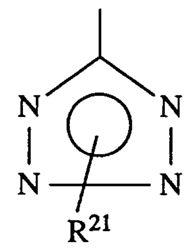

steht,
worin

| | |
|---|---|
| $R^{21}$ | Wasserstoff oder geradkettiges oder verzweigtes Alkyl mit bis zu 4 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet, |

und deren Salze.

**4.** Substituierte Mono- und Bipyridylderivate nach Anspruch 1
wobei

| | |
|---|---|
| A oder D | für ein Stickstoffatom steht und der andere Rest jeweils für die CH-Gruppe steht, |
| G, L, M und T | für die CH-Gruppe stehen, |
| V | für einen Rest der Formel |

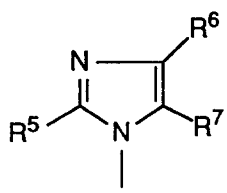 , 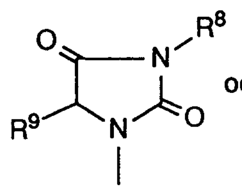 oder 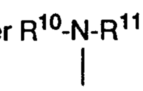

steht,
worin

| | |
|---|---|
| $R^5$, $R^8$ und $R^9$ | gleich oder verschieden sind und geradkettiges oder verzweigtes Alkyl mit bis zu 6 Kohlenstoffatomen oder Cyclopropyl bedeuten, |
| $R^6$ | Fluor, Chlor oder Brom bedeutet, |
| $R^7$ | Formyl, Carboxy, Methoxy- oder Ethoxycarbonyl oder Hydroxymethyl bedeutet, oder |
| $R^6$ und $R^7$ | unter Einbezug der Doppelbindung gemeinsam einen Pyridylrest bilden, der bis zu zweifach durch Brom oder Methyl substituiert sein kann, |
| $R^{10}$ | geradkettiges oder verzweigtes Alkyl mit bis zu 5 Kohlenstoffatomen bedeutet, oder geradkettiges oder verzweigtes Acyl mit bis zu 5 Kohlenstoffatomen bedeutet, das gegebenenfalls durch Acyl mit bis zu 5 Kohlenstoffatomen substituiert ist, |
| $R^{11}$ | Pyridyl bedeutet, das durch Carboxy oder Alkoxycarbonyl mit bis zu 3 Kohlenstoffatomen substituiert sein kann, oder geradkettiges oder verzweigtes Alkyl mit bis zu 5 Kohlenstoffatomen bedeutet, das durch Carboxy oder Alkoxycarbonyl mit bis zu 3 Kohlenstoffatomen substituiert sein kann, oder eine Gruppe der Formel $-CO-NR^{12}R^{13}$ bedeutet, worin |
| $R^{12}$ | Wasserstoff oder Methyl bedeutet |
| $R^{13}$ | geradkettiges oder verzweigtes Alkyl mit bis zu 6 Kohlenstoffatomen bedeutet, das durch Carboxy oder Alkoxycarbonyl mit bis zu 3 Kohlenstoffatomen substituiert ist, |
| $R^1$, $R^2$ und $R^3$ | für Wasserstoff stehen, |
| $R^4$ | für einen Tetrazolylrest der Formel |

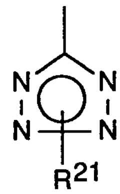

steht,
worin

$R^{21}$ Wasserstoff oder die Triphenylmethylgruppe bedeutet,

und deren Salze.

**5.** Substituierte Mono- oder Bipyridylderivate nach Anspruch 1 bis 4 zur therapeutischen Anwendung.

**6.** Verfahren zur Herstellung von substituierten Mono- oder Bipyridylderivaten nach Anspruch 1 bis 4 dadurch gekennzeichnet, daß man
Verbindungen der allgemeinen Formel (II)

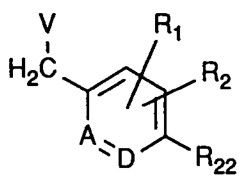

(II)

in welcher

A, D, V, $R^1$ und $R^2$ die oben angegebene Bedeutung haben

und

$R^{22}$ für eine typische Abgangsgruppe, wie beispielsweise Brom, Jod, Methan-, Toluol-, Fluor- oder Trifluormethansulfonyloxy, vorzugsweise für Brom steht,

mit Verbindungen der allgemeinen Formeln (III) oder (IIIa)

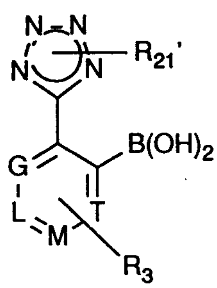

(III)

oder

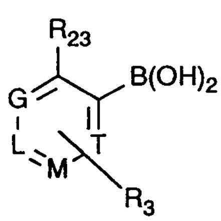

(IIIa)

in welcher

G, L, M, T und $R^3$ die oben angegebene Bedeutung haben

$R^{21'}$ für Wasserstoff oder für die Triphenylmethylgruppe steht,

und

$R^{23}$ die oben angegebene Bedeutung von $R^4$ hat, aber nicht für den Tetrazolylsubstituenten steht,

in inerten Lösemittein, in Anwesenheit einer Base und metallkatalysiert umsetzt,

und anschließend im Fall $R^{21'}$ = Triphenyimethylgruppe diese Gruppe mit Säuren in organischen Lösemitteln und/oder Wasser nach üblichen Bedingungen abspaltet,

und im Fall der Salze bevorzugt ausgehend vom freien Tetrazol ($R^{21}/R^{21'}$ = H) mit Säuren oder Basen umsetzt,

und im Fall der freien Säure $R^4$ = $CO_2H$ und des freien Tetrazols $R^{21}/R^{21'}$ = H, ausgehend von den Salzen mit Säuren umsetzt,

und gegebenenfalls im Fall der unter den Substituenten $R^4$ aufgeführten carboxylischen Reste nach Verseifung der jeweiligen Ester beispielsweise durch Amidierung oder Sulfoamidierung nach üblichen Methoden derivatisiert,

und gegebenenfalls die Substituenten nach bekannten Methoden auf jeder Verfahrensstufe variiert.

**7.** Arzneimittel enthaltend mindestens ein substituiertes Mono- oder Bipyridylderivat nach Anspruch 1 bis 4.

**8.** Arzneimittel nach Anspruch 7 zur Behandlung von Atherosklerose und arterieller Hypertonie.

**9.** Verfahren zur Herstellung von Arzneimitteln nach Anspruch 7 und 8 dadurch gekennzeichnet, daß man den Wirkstoff gegebenenfalls mit Hilfe geeigneter Hilfs- und Trägerstoffe in eine geeignete Applikationsform bringt.

**10.** Verwendung von substituierten Mono- und Bipyridylderivaten nach Anspruch 1 bis 4 zur Herstellung von Arzneimitteln.

Europäisches Patentamt

# EUROPÄISCHER RECHERCHENBERICHT

Nummer der Anmeldung
EP 94 10 8789

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X<br><br><br>D | EP-A-0 510 813 (MERCK & CO) 28. Oktober 1992<br>* das ganze Dokument *<br>& US-A-5 128 327 | 1-10 | C07D471/04<br>A61K31/435<br>C07D401/14<br>C07D401/10<br>A61K31/415<br>A61K31/44 |
| X,D | EP-A-0 518 033 (TAKEDA CHEMICAL INDUSTRIES LTD.) 16. Dezember 1992<br>* Anspruch 1; Beispiele 2,21 * | 1-10 | |
| Y | EP-A-0 499 415 (IMPERIAL CHEMICAL INDUSTRIES) 19. August 1992<br>* Seite 34, Formel XIII * | 1-10 | |
| Y,D | EP-A-0 508 393 (SEARLE & CO) 14. Oktober 1992<br>* Anspruch 1; Beispiele * | 1-10 | |
| X | BIOORGANIC & MEDICINAL CHEMISTRY LETTERS, Bd.3, Nr.6, Juni 1993, OXFORD<br>Seiten 1055 - 1060<br>D.B. REITZ ET AL. 'N1-sterically hindered 2H-imidazol-2-one angiotensin II receptor antagonists.'<br>* das ganze Dokument * | 1-10 | **RECHERCHIERTE SACHGEBIETE (Int.Cl.5)**<br>C07D |
| A | EP-A-0 533 058 (HOECHST AKTIENGESELLSCHAFT) 24. März 1993 | | |
| A | EP-A-0 400 974 (MERCK & CO) 5. Dezember 1990 | | |
| P,X | JOURNAL OF MEDICINAL CHEMISTRY, Bd.36, Nr.18, 3. September 1993, WASHINGTON US<br>Seiten 2676 - 2688<br>M. WINN ET AL. '2-(alkylamino)nicotinic acid and analogs.'<br>* Verbindung 75 * | 1-10 | |

Der vorliegende Recherchenbericht wurde für alle Patentansprüche erstellt

| Recherchenort | Abschlußdatum der Recherche | Prüfer |
|---|---|---|
| DEN HAAG | 21. September 1994 | De Jong, B |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)